# EUROPEAN PATENT SPECIFICATION

(11) **EP 0 992 238 B1**
(45) Date of publication and mention of the grant of the patent: **13.10.2004**
(21) Application number: 99100030.8
(22) Date of filing: 04.01.1999
(51) Int. Cl.: A61K 31/085, A61K 31/14, A61K 31/44

(54) **Use of triclosan for preventing and treating mucositis**
Verwendung von Triclosan zur Behandlung und Prävention von Mukositis
Utilisation de triclosan pour la prevention et le traitement de la mucosite

(30) Priority: 06.10.1998 US 167225; 05.11.1998 US 186825
(43) Date of publication of application: 12.04.2000
(73) Proprietor: I-Dent International Corporation, Bellport, New York 11713 (US)
(72) Inventor: Libin, Barry, Bellport, New York 11713 (US)
(74) Representative: Becker Kurig Straus

(56) References cited:
- EP-A- 0 528 468
- EP-A- 0 577 306
- WO-A-93/25209
- GB-A- 2 160 099
- US-A- 5 279 813

## Description

Many conditions arise which cause moderate to acute discomfort on the skin and mucous membranes. In particular, immunodeficient states may cause mucositis with accompanying inflammation; herpetic lesions may cause inflammation and fungal infections are also accompanied by inflammation.

Immunodeficient patients frequently exhibit a condition on the oral mucosa which is clinically described as oral mucositis. This condition has no known microbial or viral vector that has been implicated as the causative agent. The immunodeficiency that preceded the appearance of mucositis may arise spontaneously from genetic factors, may be caused by infections, e.g., the HIV virus or mucositis be induced as a result of chemotherapy or radiation therapy for neoplastic diseases. This condition has been difficult to treat and has not responded to treatment with antimicrobial or other agents.

The infections caused by Herpes simplex may appear anywhere on the skin or mucosa but are most often seen around the mouth, lips, conjunctiva, cornea and genitalia accompanied by an inflammatory response which causes pain and various degrees of discomfort. There are two existing types of herpes simplex virus infections, each type having multiple strains. Type 1 herpes simplex infects mucous membranes of the oral cavity as well as perioral skin, the skin above the waist and the eyes. A serious herpes simplex Type 1 infection is herpes keratitis which may result is disfunction of the cornea. Other primary herpes simplex Type 1 infections include stomatitis and dermatitis.

Herpes simplex Type 2 causes genital infections and is the second most common venereal disease not only in the United States but in many other countries.

It has been found that when the immune system is compromised as in the case of patients undergoing chemotherapy for cancer, the patients become highly susceptible to herpes simplex infections.

A large percentage of the U.S. population is affected by some form of a herpes virus infection, there being an estimated 98 million cases of herpes labialis (Type 1) occurring each year. In the case of genital herpes (Type 2) there are about 30 million cases each year.

Herpes simplex (Type 1) resides in latent form in the trigeminal ganglions in the facial area. In some individuals this virus remains inactive while in many others the virus may travel from the nerves located near the cheek bone to the lips. This gives rise to vesiculo-ulcerative eruptions around the lips, the chin and the cheeks, or under the nose.

Herpes simplex consists of evolving strains that are resistant to know anti-viral agents such as ganciclovir and acylovir. Because herpes simplex infections are not treatable by known antiviral agents, the usual protocol for such infections includes the elimination of the conditions which precipitated the viral infections and local antibiotic treatment to prevent to prevent bacterial at the site of the viral infection. But an antibiotic, such as penicillin is a bactericidal agent and as such is ineffective against a herpes simplex infection.

Unicellular fungi may cause dermal, mucosal or periodontal opportunistic infections. The incidence of such infections has risen with the increase in the number of immunocompromised patients, such as those suffering from HIV infections, transplant recipients treated with immunosuppressive drugs and patients undergoing chemotherapy and radiation therapy for cancer.

In the article entitled "In Vitro Antifungal Properties of Mouthrinses Containing Antimicrobial Agents" by Giulana et al., in J. Periodontal 1997; 68:791-801 it is noted that mouthrinses containing an antimicrobial agent, such as Triclosan or CPC might serve as an appropriate alternative to conventional antifungal drug's in the management of oral candidiasis.

Further, triclosan is described in EP-A-0 528 468 A1 as being useful as a treatment for periodontitis because it is a COX inhibitor. However, the specific inflammatory process of periodontal disease is caused by bacteria which form metabolizes.

WO 93/25209 discloses a composition that contains three absolutely required components, namely glycyrrhizin, rosemary acid and thyme oil and 6 other optional components, of which one is Irgasan OP 300 which is a tradename for triclosan and its use for affecting virus-induced infections and inflammations.

GB-A-2 160 099 A describes a treatment for athletes foot which contains urea and triclosan.

US-A-5,279,813 discloses the use of triclosan as a polymeric material as an anti-plaque composition.

EP-A-0 577 306 A1 describes a method of preventing dental plaque using a combination of triclosan and a cationic agent. This condition is caused by bacteria which elaborate the plaque-substance when the bacteria grow in the oral cavity.

It has been discovered that an alternative to conventional antifungal drugs is a composition in accordance with the invention in which a non-cationic antimicrobial agent combined synergistically with a cationic antimicrobial agent. This composite is also effective as a topical agent, in the form of an ointment or spray against superficial fungal infections.

This may include fungal infections of the head (tinea capitis), body infections (tinea corporis), "athletes foot" (tinea pedis) as well as groin and buttocks infections (tinea crusis). The composite has also been found to be effective against superficial candidiasis (moniliasis) and cutaneous candidiasis. Infections caused by candida are grouped under the term candidiasis and these infections involve the mucous membranes, scalp, skin and nails and may be accompanied by pain, itching and/or redness. The treatment of candida infections is discussed in MacNeill et al. J.Clin. Periodont. 24; 733-760 (1997), which is incorporated by reference.

The applicant has discovered a treatment for the inflammation which accompanies mucositis, heretic infections and fungal infections which is based on contacting the diseased sites on the affected area of the mucosa with triclosan alone or in combination with a cationic antimicrobial compound. The present inventor holds U.S. 5,236,699, which is incorporated by reference. That patent describes the use of a mouth rinse which contains triclosan and a cationic antimicrobial agent for use inter alia the treatment of plaque and gum diseases.

The present invention comprises a new composition f the prevention or treatment of mucositis which comprises applying to the affected area an effective amount of a composition which comprises triclosan alone or in combination with a cationic antimicrobial compound.

It is a primary object of the invention to provide a composition for treating the prevention or treatment of mucositis.

It is also an object of the invention to provide a composition for treating the inflammation of oral mucositis in immunocompromised patients.

It is also an object of the invention to provide a composition for treating mucositis using triclosan alone or in combination with a cationic antibacterial compound.

These and other objects of the invention will become apparent from a review of the appended specification.

The above-identified objects were solved by the composition according to claim 1.

The subclaims contain special embodiments of the present invention.

Mucositis is treated in accordance with the present invention by contacting the involved area with a composition which contains an amount of triclosan which is effective to treat the particular condition. Generally these compositions contain about 0.01 to 5.3wt% and preferably 0.02 to 0.5wt% of triclosan. Generally, semi-solid formulations will be formulated with higher levels of triclosan. The amount of the formulation which is applied will depend on the extent of the lesion. Generally when a liquid formulation is applied to a typical lesion, from 5ml to 30ml is applied to the lesion as a mouth rinse with the patient being instructed to eject the excess amount of the formulation from the mouth without swallowing. If a semi-solid formulation is used, then a thin film, i.e. from 0.5mm to 5mm in thickness may be applied to the affected area.

Triclosan is 2,4,4'-trichloro-2'-hydroxydiphenyl ether which is commercially available.

The triclosan is adsorbed and retained on the oral mucosa while resisting removal by saliva in the oral cavity.

The compositions may be prepared as a liquid or a semi-solid formulation. The semi-solid compositions may vary from highly viscous liquids to gels or paste like formulations.

A liquid formulation may be prepared with purified water, triclosan and a solubilizer. The solubilizer may comprise a sodium lauryl sulfate, polysorbate 20, polysorbate 40, polysorbate 60, polysorbate 80, poloxamer or mixtures thereof. The polaxamers are of the formula HO(CH₂CH₂O)ₐ(CH-(CH₃)(CH₂OH)_{b}(CH₂CH₂O)_{c}H where b is at least 15 and (CH₂CH₂O)ₐ + c is varied from 20 to 90% by weight and the weight average mol wt ranges from 10,000 to >16,000. The polyoxamers are available under the Pluronic trademark and Pluronic F127 is a preferred solubilizer. If a solubilizer is employed, it will comprise from 0.5 to 8wt% of the liquid composition. Generally, only liquid compositions in water will require a solubilizer; semi-solid formulations will not require the presence of a solubilizer.

The mucositis treating formulation may include an anti-caries agent which is soluble in water such as sodium fluoride, stannous fluoride or sodium monofluorophosphate in an amount which is effective to inhibit tooth decay in an immunocompromised patient. Generally, this amount will be from 0.01 to 4% by weight, based on the weight of the fluoride ion. The amount may be varied depending on the particular source of the fluoride ion which is chosen. Certified color may be added in a minor amount e.g. 0.1%by weight. FD&C Blue No.1 or FD&C Yellow No.5 may be used as desired.

If a cationic antibacterial agent is used in combination with the triclosan, it may be used in combination with chlorhexidine and quaternary ammonium salts such as cetylpyridinium chloride (CPC) which is the monohydrate of the quaternary ammonium salt of pyridine and cetyl chloride. CPC is cationic, highly soluble in water and alcohol. Other cationic antimicrobial agents include benzalkonium chloride, benzethonium chloride, methylbenzethonium chloride and domiphen bromide. Chlorhexidine may be applied as the free base, or as the dihydrochloride or the gluconate salt. The composition may alternatively be based on an anionic antimicrobial agent (e.g., potassium sorbate; sodium benzoate; or methyl or propyl paraben.

The combination of triclosan and the cationic antimicrobial compound has the effect that the combined agents are readily adsorbed and retained on the oral mucosa while resisting removal by saliva in the oral cavity.

The compositions may be prepared as a liquid or a semi-solid formulation. The semi-solid compositions may vary from highly viscous liquids to gels or paste like formulations.

A liquid formulation may be prepared with purified water, the triclosan, the cationic antimicrobial compound and a solubilizer. The solubilizers have been described supra.

A typical liquid formulation will comprise:

| | %weight |
|---|---|
| triclosan | 0.100 |
| CPC | 0.024 |
| Sorbitol Solution, U.S.P. | 12.000 |
| Glycerin | 10.000 |
| Sodium Saccharin, U.S.P | 0.100 |
| Pluronic FI27, NF | 4.000 |
| 190 Proof Grain Alcohol,U.S.P. | 7.000 |
| Peppermint IFL2745 | 0.152 |
| Caramel Color AP100 | 0.0085 |
| Purified water | 66.615 |

A typical fluoridated liquid formulation will comprise:

| | %weight |
|---|---|
| triclosan | 0.100 |
| CPC | 0.024 |
| Sodium Fluoride | 0.020 |
| Sorbitol Solution, U.S.P. | 11.980 |
| Glycerin | 10.000 |
| Sodium Saccharin, U.S.P | 0.100 |
| Pluronic FI27, NF | 4.000 |
| 190 Proof Grain Alcohol,U.S.P. | 7.000 |
| Peppermint IFL2745 | 0.152 |
| Caramel Color AP100 | 0.0085 |
| Purified water | 66.615 |

A typical semisolid formulation which is a cream: will include:

| | |
|---|---|
| triclosan | 0.1-5.3wt% |
| Cetaryl glucoside and cetaryl alcohol (Emulgade PL 68/50, Henkel) | 0.5-6.7wt% |
| Cetaryl alcohol (Lanette, Henkel) | 0.5-7.7wt% |
| Coco-Caprylate (Cedol LC, Henkel) | 0.5-6.0wt% |
| Dicapryl ether (Cetiet, Henkel) | 0.25-5.0wt% |
| Sweet almond oil | 0.25-5.0wt% |
| Petrolatum | 0.5-6.0wt% |
| Dimethicone (Silicone DC 200CS/Dow) | 0.1-5wt% |

| Phase B | |
|---|---|
| CPC | 0.01-4.4wt% |
| glycerin | 0.5-4.6wt% |
| Sodium methylparaben/Sodium paraben | 0.01-0.03wt% |
| or | |
| Sodium benzoate | 0.25-0.3wt% |
| Deionized water | 10-90wt% |

An example of a semi-solid formulation according to the invention is as follows:

| Phase A | |
|---|---|
| triclosan | 0.3wt% |
| Cetaryl glucoside and cetaryl alcohol (Emulgade PL 68/50, Henkel) | 3.7wt% |
| Cetaryl alcohol (Lanette, Henkel) | 3.7wt% |
| Coco-Caprylate (Cedol LC, Henkel) | 3.0wt% |
| Dicapryl ether (Cetiet, Henkel) | 2.0wt% |
| Sweet almond oil | 2.0wt% |
| Petrolatum | 3.0wt% |
| Dimethicone (Silicone DC 200CS/Dow) | 0.6wt% |

| Phase B | |
|---|---|
| CPC | 0.1wt% |
| Glycerin | 2.6wt% |
| Sodium methylparaben | 0.18wt% |
| sodium paraben | 0.02wt% |
| | |
| Deionized water to | 100.0wt% |

| Phase C | |
|---|---|
| Tocophenyl acetate (cophenol 1260/Henkel) | 1.0wt% |

The composition is prepared by separately heating Phase A and Phase B to 80°C prior to forming these phases. Phase C is added with stirring at 55°C until a smooth homogeneous mixture is obtained.

The foregoing description of a preferred embodiment of the invention has been presented for purposes of illustration and description. It is not intended to be exhaustive or to limit the invention to the precise form disclosed. Obvious modifications or variations are possible in light of the above teachings. All such obvious modifications and variations are intended to be within the scope of the appended claims. A typical liquid formulation will comprise:

| | %weight |
|---|---|
| triclosan | 0.100 |
| Sorbitol Solution, U.S.P. | 12.000 |
| Glycerin | 10.000 |
| Sodium Saccharin, U.S.P | 0.100 |
| Pluronic FI27, NF | 4.000 |
| 190 Proof Grain Alcohol,U.S.P. | 7.000 |
| Peppermint IFL2745 | 0.152 |
| Caramel Color AP100 | 0.0085 |
| Purified water | 66.639 |

A typical fluoridated liquid formulation will comprise:

| | %weight |
|---|---|
| triclosan | 0.100 |
| Sodium Fluoride | 0.020 |
| Sorbitol Solution, U.S.P. | 11.980 |
| Glycerin | 10.000 |
| Sodium Saccharin, U.S.P | 0.100 |
| Pluronic FI27, NF | 4.000 |
| 190 Proof Grain Alcohol,U.S.P. | 7.000 |
| Peppermint IFL2745 | 0.152 |
| Caramel Color AP100 | 0.0085 |
| Purified water | 66.639 |

A typical semisolid formulation which is a cream: will include:

| | |
|---|---|
| triclosan | 0.1-5.3wt% |
| Cetaryl glucoside and cetaryl alcohol (Emulgade PL 68/50, Henkel) | 0.5-6.7wt% |
| Cetaryl alcohol (Lanette, Henkel) | 0.5-7.7wt% |
| Coco-Caprylate (Cedol LC, Henkel) | 0.5-6.0wt% |
| Dicapryl ether (Cetiet, Henkel) | 0.25-5.0wt% |
| Sweet almond oil | 0.25-5.0wt% |
| Petrolatum | 0.5-6.0wt% |
| Dimethicone (Silicone DC 200CS/Dow) | 0.1-5wt% |

| Phase B | |
|---|---|
| glycerin | 0.5-4.6wt% |
| Sodium methylparaben/Sodium paraben | 0.01-0.03wt% |
| or | |
| Sodium benzoate | 0.25-0.3wt% |
| Deionized water | 10-90wt% |

An example of a semi-solid formulation according to the invention is as follows:

| Phase A | |
|---|---|
| triclosan | 0.3wt% |
| Cetaryl glucoside and cetaryl alcohol (Emulgade PL 68/50, Henkel) | 3.7wt% |
| Cetaryl alcohol (Lanette, Henkel) | 3.7wt% |
| Coco-Caprylate (Cedol LC, Henkel) | 3.0wt% |
| Dicapryl ether (Cetiet, Henkel) | 2.0wt% |
| Sweet almond oil | 2.0wt% |
| Petrolatum | 3.0wt% |
| Dimethicone (Silicone DC 200CS/Dow) | 0.6wt% |

| Phase B | |
|---|---|
| Glycerin | 2.6wt% |
| Sodium methylparaben | 0.18wt% |
| Sodium paraben | 0.02wt% |
| | |
| Deionized water to | 100.0wt% |

| Phase C | |
|---|---|
| Tocophenyl acetate (cophenol 1260/Henkel) | 1.0wt% |

The composition is prepared by separately heating Phase A and Phase B to 80°C prior to forming these phases. Phase C is added with stirring at 55°C until a smooth homogeneous mixture is obtained.

The foregoing description of a preferred embodiment of the invention has been presented for purposes of illustration and description. Obvious modifications or variations are possible in light of the above teachings.

## Claims

1. Use of triclosan or a combination of triclosan and a cationic antimicrobial compound for the production of a composition for the treatment of mucositis.

2. Use of triclosan according to claim 1 for the production of a composition for the treatment of mucositis in an immunocompromised patient.

3. Use according to claims 1 or 2 wherein triclosan is combined in a liquid formulation with a solubilizer.

4. Use according to claims 1 or 2 wherein the triclosan is in a semi-solid formulation.

5. Use according to anyone of the proceeding claims 1 to 4 wherein the composition includes a fluoride.

6. Use of triclosan for the production of a composition for treatment of mucositis in an immunocompromised patient according to anyone of the proceeding claims 1 to 5, said composition comprising a composition which comprises triclosan in an amount which is effective to alleviate the symptoms of mucositis and an amount of a fluoride compound which is effective to inhibit tooth decay in an immunocomprised patient.

7. Use according to anyone of the proceeding claims 1 to 6, wherein said composition comprise triclosan and a cationic antibacterial agent in amounts which are effective to alleviate the symptoms of mucositis.

8. Use according to claim 7 wherein the cationic agent is selected from the group consisting of chlorhexidine, cetylpyridium chloride, benzalkonium chloride, benzethonium chloride, methylbenzethonium chloride and domiphen bromide.

9. Use according to claims 7 or 8 wherein the cationic agent is cetylpyridium chloride.

10. Use according to claim 9 wherein the triclosan and cationic agent are combined in a liquid formulation with a solubilizer.

11. Use according to claim 9 wherein the triclosan and the cationic agent are combined in a semi-solid formulation.

## Patentansprüche

1. Verwendung von Triclosan oder einer Kombination von Triclosan und einer kationischen, antimikrobiellen Verbindung zur Herstellung einer Zusammensetzung zur Behandlung von Mukositis.

2. Verwendung von Triclosan gemäß Anspruch 1 zur Herstellung einer Zusammensetzung zur Behandlung von Mukositis in einem abwehrgeschwächten Patienten.

3. Verwendung gemäß Ansprüchen 1 oder 2, wobei Triclosan in einer flüssigen Formulierung mit einem Lösungsvermittler kombiniert ist.

4. Verwendung gemäß Ansprüchen 1 oder 2, wobei das Triclosan in einer halbfesten Formulierung ist.

5. Verwendung gemäß irgendeinem der vorangehenden Ansprüche 1 bis 4, wobei die Zusammensetzung ein Fluorid enthält.

6. Verwendung von Triclosan zur Herstellung einer Zusammensetzung zur Behandlung von Mukositis in einem abwehrgeschwächten Patienten gemäß irgendeinem der vorangehenden Ansprüche 1 bis 5, wobei die Zusammensetzung eine Zusammensetzung enthält, die Triclosan in einer wirksamen Menge enthält, um die Symptome von Mukositis zu lindern, und eine wirksame Menge einer Fluoridverbindung enthält, um Zahnkaries in einem abwehrgeschwächten Patienten zu hemmen.

7. Verwendung gemäß irgendeinem der vorangehenden Ansprüche 1 bis 6, wobei die Zusammensetzung Triclosan und ein kationisches, antibakterielles Mittel in wirksamen Mengen enthält, um die Symptome von Mukositis zu lindern.

8. Verwendung gemäß Anspruch 7, wobei das kationische Mittel ausgewählt ist aus der Gruppe, bestehend aus Chlorhexidin, Cetylpyridiniumchlorid, Benzalkoniumchlorid, Benzethoniumchlorid, Methylbenzethoniumchlorid und Domiphenbromid.

9. Verwendung gemäß Ansprüchen 7 oder 8, wobei das kationische Mittel Cetylpyridiniumchlorid ist.

10. Verwendung gemäß Anspruch 9, wobei das Triclosan und das kationische Mittel in einer flüssigen Formulierung mit einem Lösungsvermittler kombiniert sind.

11. Verwendung gemäß Anspruch 9, wobei das Triclosan und das kationische Mittel in einer halbfesten Formulierung kombiniert sind.

## Revendications

1. Utilisation de triclosan ou d'une combinaison de triclosan et d'un composé cationique antimicrobien pour la production d'une composition destinée au traitement de la mucosite.

2. Utilisation de triclosan selon la revendication 1 pour la production d'une composition pour le traitement de la mucosite chez un patient immunodéprimé.

3. Utilisation selon les revendications 1 ou 2, dans laquelle le triclosan est combiné dans une formulation liquide avec un agent solubilisant.

4. Utilisation selon les revendications 1 ou 2, dans laquelle le triclosan est une formulation semi-solide.

5. Utilisation selon l'une quelconque des revendications précédentes 1 à 4, dans laquelle la composition comprend un composé fluoré.

6. Utilisation de triclosan pour la production d'une composition pour le traitement de la mucosite chez un patient immunodéprimé selon l'une quelconque des revendications précédentes 1 à 5, ladite composition comprenant une composition qui comprend le triclosan en une quantité efficace pour soulager les symptômes de la mucosite et en une quantité de composé fluoré efficace pour inhiber les caries dentaires chez un patient immunodéprimé.

7. Utilisation selon l'une quelconque des revendications précédentes 1 à 6, dans laquelle ladite composition comprend le triclosan et un agent cationique antibactérien en quantités efficaces pour soulager les symptômes de la mucosite.

8. Utilisation selon la revendication 7, dans laquelle l'agent cationique est choisi dans le groupe constitué de la chlorhexidine, du chlorure de cétypyridium, du chlorure de benzalkonium, du chlorure de benzéthonium, du chlorure de méthylbenzéthonium et du bromure de domiphène.

9. Utilisation selon les revendications 7 ou 8, dans laquelle l'agent cationique est le chlorure de cétylpyridium.

10. Utilisation selon la revendication 9, dans laquelle le triclosan et l'agent cationique sont combinés dans une formulation liquide avec un agent solubilisant.

11. Utilisation selon la revendication 9, dans laquelle le triclosan et l'agent cationique sont combinés dans une formulation semi-solide.
